(19) Europäisches Patentamt
European Patent Office
Office européen des brevets

(11) Veröffentlichungsnummer: **0 069 918 B1**

# (12) EUROPÄISCHE PATENTSCHRIFT

(45) Veröffentlichungstag der Patentschrift: **10.04.85**

(51) Int. Cl.⁴: **C 25 B 3/04,** C 07 D 315/00

(21) Anmeldenummer: **82105808.8**

(22) Anmeldetag: **30.06.82**

(54) **Verfahren zur Herstellung von Cyclopentadecanolid.**

(30) Priorität: **10.07.81 DE 3127242**

(43) Veröffentlichungstag der Anmeldung:
**19.01.83 Patentblatt 83/3**

(45) Bekanntmachung des Hinweises auf die Patenterteilung:
**10.04.85 Patentblatt 85/15**

(84) Benannte Vertragsstaaten:
**BE CH DE FR GB IT LI NL**

(56) Entgegenhaltungen:
**SU - A - 521 274**

**CHEMICAL ABSTRACTS, Band 95, Nr. 3, 20. Juli 1981, Seite 679, Nr. 24858y, Columbus, Ohio, USA**
**ELECTROCHIMICA ACTA, Band 26. Nr. 7, Juli 1981, Seiten 819-820, Pergamon Press Ltd., Oxford, G.B. D. PLETCHER et al.: "The reduction of acetophenone to ethylbenzene at a platinised platinum electrode"**
**M.M. BAIZER: "Organic electrochemistry", 1973, Marcel Dekker, Inc., Seiten 367-371, New York, USA**

(73) Patentinhaber: **BASF Aktiengesellschaft, Carl-Bosch-Strasse 38, D-6700 Ludwigshafen (DE)**

(72) Erfinder: **Degner, Dieter, Dr., Kurpfalzstrasse 8, D-6701 Dannstadt-Schauernheim (DE)**
Erfinder: **Hoffmann, Werner, Dr., Ringstrasse 11 C, D-6708 Neuhofen (DE)**
Erfinder: **Thoemel, Frank, Dr., Leberstrasse 17, D-6940 Weinheim (DE)**

**EP 0 069 918 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von Cyclopentadecanolid durch Elektroreduktion von 12-Oxo-cyclopentadecanolid.

Moschus-Riechstoffe sind von fundamentaler Bedeutung für die Parfümerie. Cyclopentadecanolid, ein in der Natur in der Angelikapflanze vorkommender Moschusriechstoff, besitzt eine der feinsten und in der Parfümerie am meisten geschätzten Moschusnoten. Es hat daher nicht an Versuchen gefehlt, Cyclopentadecanolid technisch herzustellen.

So wurde z.B. versucht, 15-Hydroxi- oder 15-Halogen-pentadecansäure zu Cyclopentadecanolid zu cyclisieren [vgl. z.B. M.H. Klowen, J.G.J. Kok, Parfümerie und Kosmetik 43, 35 (1962); G. Ohloff, La France et ses parfums 1970, 146; S. Abe, T. Eto, J. Tsujito, Cosmetics and Perfumery 88, 67 (1973)]. Die Cyclisierung wird nach dem Ruggli-Zieglerschen Verdünnungsprinzip durchgeführt. Die Raum-Zeit-Ausbeuten sind daher sehr unbefriedigend. Weiterhin sind die benötigten substituierten Pentadecansäurederivate nur über aufwendige mehrstufige Synthesen zugänglich. In der DE-AS 2 731 543 und den darin angegebenen Literaturstellen werden Synthesen von Cyclopentadecanolid beschrieben, die von 13-Oxa-bicyclo-[10.4.0]-hexadecen-[1(12)] ausgehen. Hierbei sind die häufig nicht befriedigenden Ausbeuten oder die Notwendigkeit des Einsatzes von Peroxiden von Nachteil. Auch die Überführung in ein Oximinolakton, anschliessende reduktive Ringöffnung zu 15-Hydroxypentadecansäure und schliesslich thermischer Ringschluss zu Cyclopentadecanolid stellt einen sehr aufwendigen und technisch wenig befriedigenden Syntheseweg dar.

12-Oxocyclopentadecanolid wäre ein geeignetes Vorprodukt für die Synthese von Cyclopentadecanolid, wenn es gelänge, die Carbonylgruppe in 12-Stellung selektiv zur Methylengruppe zu reduzieren. Alle bisherigen Bemühungen in dieser Richtung, wie die Clemmensen-Reduktion mit Zn-amalgam (SU-PS 521 274) oder die Umwandlung in ein Tosylhydrazon mit anschliessender Fragmentierung (J.R. Mahajan, H. Aranjo, Synthesis 1980, 64) haben jedoch nur mässige Ausbeuten gebracht.

Aus M.M. Baizer: Organic Electrochemistry, 1973, Marcel Decker, Inc., New York, USA, Seite 370 ist es bekannt, dass die Ketogruppe in Ketoestern gleichzeitig mit dem Sauerstoff der Estergruppe unter Eliminierung der Alkoxygruppe reduziert wird.

Es wurde nun gefunden, dass man Cyclopentadecanolid der Formel

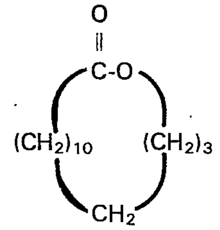

I

besonders vorteilhaft dadurch herstellen kann, dass man 12-Oxocyclopentadecanolid der Formel

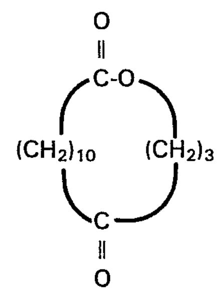

II

elektrochemisch reduziert.

Die elektrochemische Reduktion wird in den technisch üblichen Elektrolysezellen durchgeführt. Als Beispiel sei eine geteilte Zelle nach Bauart einer Filterpresse genannt. Als Diaphragmen lassen sich beispielsweise Kationenaustauschermembranen einsetzen. Als Elektrolyt dient bei dem erfindungsgemässen Verfahren eine Lösung von II in oder eine Emulsion von II mit einem leitfähigen System. Beispielsweise wird II in einem Alkohol, Ether oder Kohlenwasserstoff gelöst. Als Alkohole seien z.B. Methanol, Ethanol, Isopropanol oder Butanol genannt; als Ether können z.B. Dioxan, Tetrahydrofuran oder Di- bzw. oligomere Glykole eingesetzt werden. Als Kohlenwasserstoffe kommen z.B. Toluol oder Xylol in Betracht. Als leitfähige Systeme werden bevorzugt Säuren eingesetzt, die Wasser enthalten können. Als Beispiele seien Schwefelsäure, Salzsäure, Sulfonsäuren oder $HBF_4$ genannt. Als Kathodenmaterialien kommen z.B. Platin oder andere Edelmetalle, Quecksilber, Blei, Nickel, Zink und Graphit in Betracht. Bevorzugtes Kathodenmaterial ist Cadmium. Die Elektrolyse wird beispielsweise bei Stromdichten von 1 bis 50 $A/dm^2$, insbesondere bei 3 bis 10 $A/dm^2$ durchgeführt. Die Temperaturen bei der Elektrolyse liegen beispielsweise zwischen —20 und +95°C. Der Umsatz von II kann in weiten Grenzen gewählt werden. Man arbeitet bevorzugt so, dass II zu über 30% umgesetzt wird. Unumgesetztes II kann zur Elektrolyse zurückgeführt werden. Die Aufarbeitung der Elektrolyseausträge erfolgt nach bekannten Methoden, z.B. durch Destillation oder Extraktion. Das Verfahren kann sowohl diskontinuierlich als auch kontinuierlich durchgeführt werden.

Das erfindungsgemässe Verfahren wird an folgenden Beispielen weiter verdeutlicht.

*Beispiel 1 bis 7*

Man führt die Elektrolyse in einer geteilten Zelle mit Kationenaustauschermembran durch. Die Elektrodenfläche beträgt 1 $dm^2$. Als Anode wird eine $PbO_2$-Elektrode verwendet. Der Anolyt besteht aus 30%iger Schwefelsäure. Die Elektrolyse wird mit 6 F/Mol II durchgeführt. Weitere Einzelheiten der Versuche sind in der nachfolgenden Tabelle zusammengestellt. Sowohl der Katholyt als auch der Anolyt werden während der Elektrolyse über je einen Wärmetauscher umgepumpt. Nach Beendigung der Elektrolyse wird der Katholyt auf 1,5 l Eiswasser gegeben und anschliessend je zweimal mit Methylenchlorid und Methyl-tert.-butylether extrahiert. Die organischen Phasen werden vereinigt, mit Natriumbicarbonat neutral gewaschen und getrocknet. Dann werden die Lösungsmittel abdestilliert. Der Rückstand wird gewogen und der Gehalt an II und I gaschromatographisch ermittelt. Hieraus werden Umsatz von II und Ausbeute an I berechnet. Die Ergebnisse sind ebenfalls in nachfolgender Tabelle zusammengestellt.

*Elektrosynthese von Cyclopentadecanolid*

| Beispiel | Kathode | Katholyt | T (°C) | J ($A/dm^2$) | Uz (V) | Umsetzungsprodukt Menge (g) | Gehalt II (%) | GC I (%) | Umsatz II (%) | Ausbeute I (%) |
|---|---|---|---|---|---|---|---|---|---|---|
| 1 | Cd | 77 g II<br>684 g $CH_3OH$<br>77 g $H_2SO_4$, 30% | 17-20 | 5 | 10.5-13.0 | 67.5 | 20.3 | 50.6 | 82 | 58 |
| 2 | Cd | 77 g II<br>884 g Dioxan<br>300 g $H_2SO_4$, 30% | 24-25 | 5 | 9.0-13.2 | 72.0 | 23.5 | 70.6 | 78 | 90 |
| 3 | Cd | 77 g II<br>684 g $CH_3OH$<br>77 g $HBF_4$, 35% | 24-25 | 5 | 7.0-10.0 | 62.1 | 32.9 | 61.8 | 74 | 72 |
| 4 | Zn | 77 g II<br>684 g $CH_3OH$<br>77 g $H_2SO_4$, 30% | 23 | 5 | 12.6 | 70.8 | 72.2 | 27.8 | 34 | 81 |
| 5 | Pb | 77 g II<br>684 g $CH_3OH$<br>77 g $H_2SO_4$, 30% | 20-22 | 5 | 13.8-14.0 | 72.0 | 82.2 | 16.2 | 23 | 70 |
| 6 | Hg | 12.6 g II<br>112 g $CH_3OH$<br>12.6 g $H_2SO_4$, 30% | 18-19 | 2.7 | 14.0-19.0 | 12.0 | 90.8 | 5.0 | 13 | 37 |
| 7 | Pt | 77 g II<br>684 g $CH_3OH$<br>77 g $H_2SO_4$, 30% | 21-24 | 5 | 12.8-13.2 | 71.8 | 91.9 | 4.1 | 14 | 28 |

J: Stromdichte T: Temperatur Elektrolyse Uz: Zellspannung
II: 12-Oxo-cyclopentadecanolid I: Cyclopentadecanolid

**Patentansprüche**

1. Verfahren zur Herstellung von Cyclopentadecanolid der Formel I

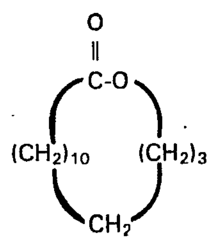

I

dadurch gekennzeichnet, dass man 12-Oxo-cyclopentadecanolid der Formel

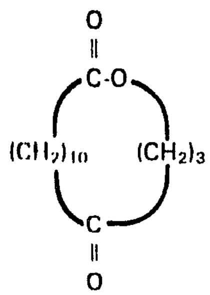

II

elektrochemisch reduziert.

2. Verfahren nach Anspruch 1, dadurch gekennzeichnet, dass man die Elektroreduktion in einem sauren Elektrolyten durchführt.

3. Verfahren nach Anspruch 1, dadurch gekennzeichnet, dass man die Elektroreduktion an Cadmiumelektroden durchführt.

4. Verfahren nach Anspruch 1, dadurch gekennzeichnet, dass man die Elektrolyse bei Stromdichten von 1 bis 50 $A/dm^2$ und Temperaturen von —20 bis +95°C durchführt.

**Claims**

1. A process for the preparation of cyclopentadecanolide of the formula I

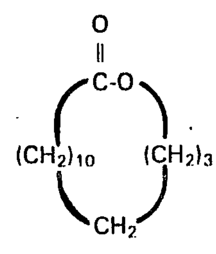

I

wherein 12-oxocyclopentadecanolide of the formula

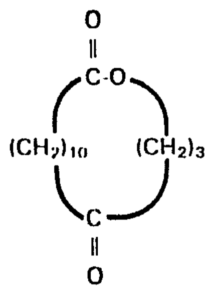

II

is reduced electrochemically.

2. A process as claimed in claim 1, wherein the electrochemical reduction is carried out in an acid electrolyte.

3. A process as claimed in claim 1, wherein the electrochemical reduction is carried out on a cadmium electrode.

4. A process as claimed in claim 1, wherein the electrolysis is carried out at a current density of from 1 to 50 A/dm$^2$ and at from —20 to +95°C.

## Revendications

1. Procédé pour la production de cyclopentadécanolide de formule I

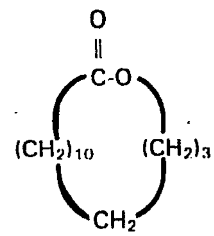

I

caractérisé en ce qu'on réduit électrochimiquement un 12-oxocyclopentadécanolide de formule

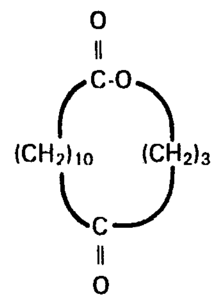

II

2. Procédé selon la revendication 1, caractérisé en ce qu'on effectue la réduction électrolytique dans un électrolyte acide.

3. Procédé selon la revendication 1, caractérisé en ce qu'on effectue la réduction électrolytique sur des électrodes de cadmium.

4. Procédé selon la revendication 1, caractérisé en ce qu'on effectue l'électrolyse avec des densités de courant de 1 à 50 A/dm$^2$ et à des températures de —20 à +95°C.